(19) Europäisches Patentamt
European Patent Office
Office européen des brevets

(11) Veröffentlichungsnummer : **0 150 015**
**B1**

(12) # EUROPÄISCHE PATENTSCHRIFT

(45) Veröffentlichungstag der Patentschrift : **16.06.87**

(51) Int. Cl.[4] : **C 07 C 103/737**, A 61 K 31/16

(21) Anmeldenummer : **85100171.9**

(22) Anmeldetag : **09.01.85**

(54) **Norbornan- und Norbornencarbonsäureamide und Verfahren zu deren Herstellung.**

(30) Priorität : **20.01.84 DE 3401949**

(43) Veröffentlichungstag der Anmeldung : **31.07.85 Patentblatt 85/31**

(45) Bekanntmachung des Hinweises auf die Patenterteilung : **16.06.87 Patentblatt 87/25**

(84) Benannte Vertragsstaaten : **AT BE CH DE FR GB IT LI NL SE**

(56) Entgegenhaltungen :
**US-A- 3 636 094**
**US-A- 4 324 912**

(73) Patentinhaber : **BAYER AG**
**Konzernverwaltung RP Patentabteilung**
**D-5090 Leverkusen 1 Bayerwerk (DE)**

(72) Erfinder : **Lieb, Folker, Dr.**
**Alfred-Kubin-Strasse 1**
**D-5090 Leverkusen 1 (DE)**
Erfinder : **Oediger, Hermann, Dr.**
**Roggendorfstrasse 51**
**D-5000 Koeln 80 (DE)**
Erfinder : **Kabbe, Hans-Joachim, Dr.**
**Walter-Flex-Strasse 16**
**D-5090 Leverkusen 1 (DE)**
Erfinder : **Niewöhner, Ulrich, Dr.**
**Gartenstrasse 3**
**D-5632 Wermelskirchen 3 (DE)**
Erfinder : **Perzborn, Elisabeth, Dr.**
**Am Tescher Busch 13**
**D-5600 Wuppertal 11 (DE)**
Erfinder : **Seuter, Friedel, Dr.**
**Moospfad 16**
**D-5600 Wuppertal 1 (DE)**



Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Norbornan- und Norbornencarbonsäureamide und Verfahren zu ihrer Herstellung.

Thrombose und arteriosklerotische Gefäßveränderungen werden vor allem durch die Wechselwirkung zweier Metaboliten der Arachidonsäure, nämlich durch das Thromboxan $A_2$ ($TXA_2$) und durch das Prostacyclin ($PGI_2$) gesteuert. $TXA_2$ wirkt auf die Blutplättchen aggregierend, und $PGI_2$ hat eine antiaggregierende Wirkung. Darüber hinaus wirkt $TXA_2$ vasonkonstriktorisch und $PGI_2$ vasodilatatorisch.

Bei einer Reihe von thrombo-embolischen und ischämischen Erkrankungen führt Hyperaggregabilität der Plättchen bzw. ein erhöhter Plättchenverbrauch zu einer gesteigerten Thromboxansynthese, so daß das $TXA_2$- und $PGI_2$-Gleichgewicht gestört ist. Es ist deshalb zur Therapie und Prophylaxe von thrombo-embolischen und ischämischen Erkrankungen wünschenswert, die Thromboxanwirkung zu hemmen und damit die protektive Eigenschaft des $PGI_2$ zu erhöhen.

Es wurde nunmehr überraschend gefunden, daß bestimmte Norbornan- und Norbornencarbonsäureamide eine spezifische und starke antagonistische Wirkung bezüglich Thromboxan $A_2$ aufweisen.

Gefunden wurden Thromboxan-antagonistische und plättchenaggregationshemmende Norbornanbeziehungsweise Norbornencarbonsäureamide der allgemeinen Formel (I),

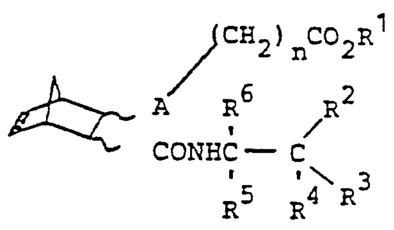

(I)

in welcher

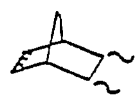

für die Teilstrukturen

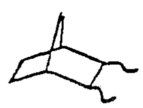

oder

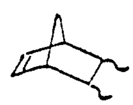

steht,

A für eine

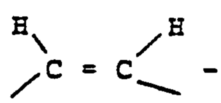

oder $-CH_2CH_2$-Gruppe steht,

$R^1$ und $R^2$ für Wasserstoff oder einen Alkylrest mit 1-6 Kohlenstoffatomen stehen,

$R^3$ für ein gegebenenfalls durch Halogen oder Alkyl mit 1 bis 2 Kohlenstoffatomen substituiertes Alkyl oder Alkenyl mit 1-8 Kohlenstoffatomen oder für ein Cycloalkyl und Cycloalkenyl mit 3 bis 7 Kohlenstoffatomen oder einen gegebenenfalls durch Halogen, Alkyl mit 1 bis 2 Kohlenstoffatomen oder Halogenalkyl mit 1 bis 2 Kohlenstoffatomen substituierten Aromaten mit 6 oder 10 Kohlenstoffatomen oder für einen fünf- oder sechsgliedrigen Heteroaromaten steht,

$R^4$ für Wasserstoff oder eine Hydroxygruppe steht, $R^5$ und $R^6$ gleich oder verschieden sind und für Wasserstoff oder Alkyl mit 1 bis 4 Kohlenstoffatomen stehen,

n für die Zahlen 2 bis 6 steht und falls $R^1$ Wasserstoff bedeutet auch deren physiologisch verträgliche Salze.

Die Schreibweise ~ bedeutet, daß die Seitenketten am Bicyclus exo- oder endo-ständig sind.

Bevorzugt sind Verbindungen, in denen $R_1$ für Wasserstoff, Methyl oder Ethyl steht,

Verbindungen, in denen n für 2-4 steht,

Verbindungen, in denen $R^4$ für Wasserstoff oder eine Hydroxygruppe steht, und

Verbindungen, in denen $R^5$ und $R^6$ für Wasserstoff stehen.

Die neuen Norbornan- und Norbornencarbonsäureamide können sowohl als Enantiomere, Enantiomerenpaare und Diastereomerenpaare vorliegen.

Es wurde ferner gefunden, daß man die Norbornan- und Norbornencarbonsäureamide der allgemeinen Formel (I) erhält, wenn man gemäß dem Reaktionsschema I ein Lacton der allgemeinen Formel (II)

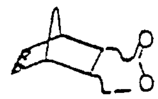

(II)

in welcher

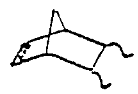

für die Teilstrukturen

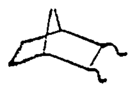

oder

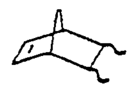

steht, zu einem Lactol der allgemeinen Formel (III)

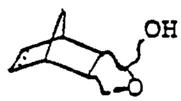

(III)

in welcher

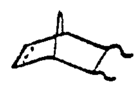

die obige Bedeutung hat, mit einem aluminium-organischen Hydrid reduziert und das so erhaltene Lactol mit einem Phosphoniumsalz der allgemeinen Formel (IV)

$$[(R^7)_3\overset{+}{P}(CH_2)_{n+1}CO_2H]X^- \qquad (IV)$$

in welcher
- $R^7$ für einen Arylrest,
- X für ein Halogen und
- n für eine Zahl zwischen 2 und 6 steht,

in Anwesenheit einer Base zu einer Säure der allgemeinen Formel (V)

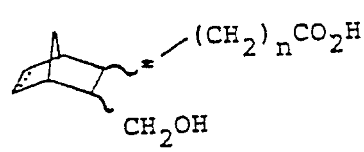

(V)

in welcher

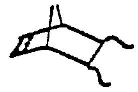

und n die obigen Bedeutungen haben, umsetzt und anschließend diese mit einem Alkohol der allgemeinen Formel (VI)

$$R^8OH \qquad (VI)$$

in welcher $R^8$ für eine Alkylrest mit 1 bis 6 Kohlenstoffatomen steht, zu dem Ester der allgemeinen Formel (VII)

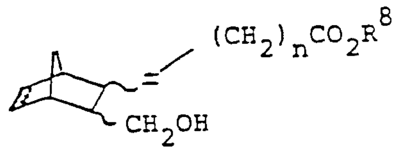

(VII)

in welcher

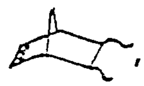

n und $R^8$ die oben angegebenen Bedeutungen haben, umsetzt und, falls in der allgemeinen Formel (I) A für

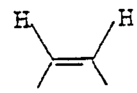

steht, diese zu einer Säure der allgemeinen Formel (VIII)

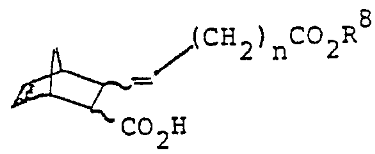

(VIII)

in welcher

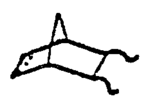

n und $R^8$ die obigen Bedeutungen haben, oxidiert und die so erhaltene Säure gegebenenfalls unter Überführung in einen aktivierten Ester mit einem Amin der allgemeinen Formel (IX)

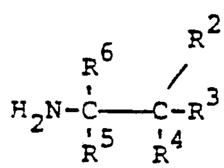

(IX)

in welcher

$R^2$ für Wasserstoff oder einen Alkylrest mit 1-6 Kohlenstoffatomen steht,

$R^3$ für ein gegebenenfalls durch Halogen oder Alkyl mit 1 bis 2 Kohlenstoffatomen substituiertes Alkyl oder Alkenyl mit 1 bis 8 Kohlenstoffatomen oder für ein Cycloalkyl und Cycloalkenyl mit 3 bis 7 Kohlenstoffatomen oder einen gegebenenfalls durch Halogen, Alkyl mit 1 bis 2 Kohlenstoffatomen oder Halogenalkyl mit 1 bis 2 Kohlenstoffatomen substituierten Aromaten mit 6 oder 10 Kohlenstoffatomen oder für einen fünf- oder sechsgliedrigen Heteroaromaten steht,

$R^4$ für Wasserstoff oder eine Hydroxygruppe steht,

$R^5$ und $R^6$ gleich oder verschieden sind und für Wasserstoff oder Alkyl mit 1 bis 4 Kohlenstoffatomen stehen,

zu den Amid-Estern (I) oder gegebenenfalls unter nachfolgender Verseifung zu den Amid-Säuren (I) umsetzt, oder falls in der allgemeinen Formel (I) A für eine $—CH_2CH_2$-Gruppe und

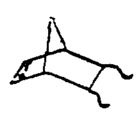

für die Teilstruktur

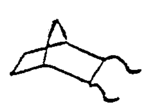

steht, den ungesättigten Ester der allgemeinen Formel (VII) zu dem Ester der allgemeinen Formel (X)

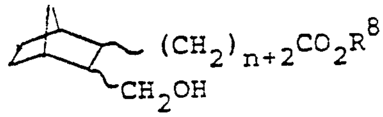

(X)

in welcher $R^8$ und n die obengenannten Bedeutungen haben, katalytisch hydriert, diesen zu einer Säure der allgemeinen Formel (XI),

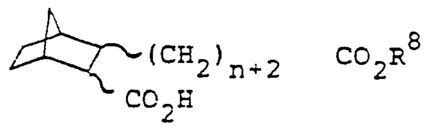

(XI)

in welcher $R^8$ und n die obigen Bedeutungen besitzen, umsetzt und die erhaltene Säure (XI) gegebenenfalls unter Überführung in einen aktivierten Ester mit einem Amin der allgemeinen Formel (IX) zu den Amid-Estern (I) oder gegebenenfalls unter nachfolgender Verseifung zu den Amid-Säuren (I) umsetzt.

Verwendet man in der ersten Stufe des Verfahrens als Ausgangsstoff Hexahydro-4,7-methano-isobenzofuran-1(3H)-on und als Reduktionsmittel Natrium-bis(2-methoxy-ethoxy)-dihydrido-aluminat, in der zweiten Stufe 4-Carboxy-butyl-triphenylphosphoniumbromid und als Base Natriumhydrid in Dimethylsulfoxid, in der dritten Stufe Methanol in Gegenwart einer starken Säure, in der vierten Stufe Pyridiniumdichromat, in der fünften Stufe 2-Hydroxy-2-phenyl-propylamin und Dicyclohexylcarbodiimid, in der sechsten Stufe Natronlauge und in einer Variante des erfindungsgemäßen Verfahrens als Hydrierungsmittel Wasserstoff und als Katalysator Palladium auf Kohle, als Oxidationsmittel in der nachfolgenden Stufe Pyridiniumdichromat und als Amin in einer weiteren nachfolgenden Stufe 2-(m-Chlorphenyl)-2-hydroxyethylamin, so lassen sich die Reaktionsabläufe durch folgendes Formelschema wiedergeben :

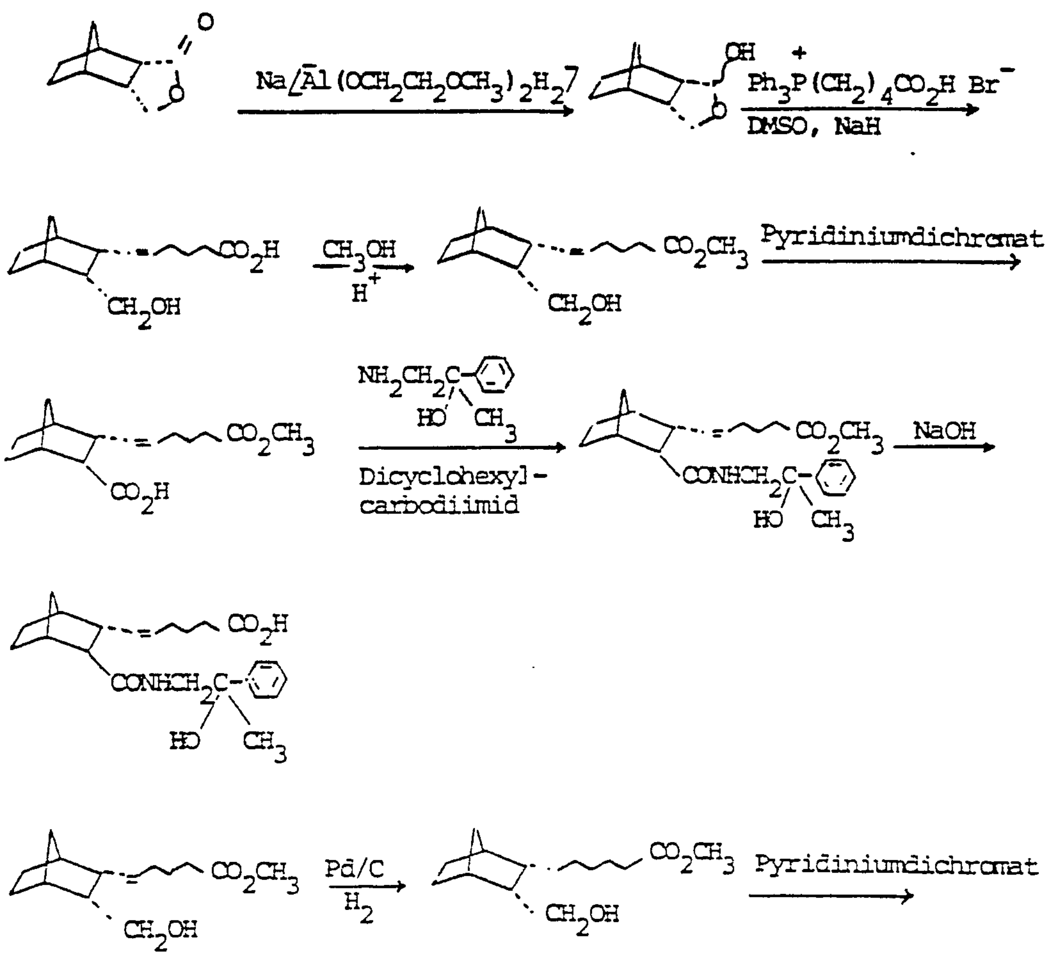

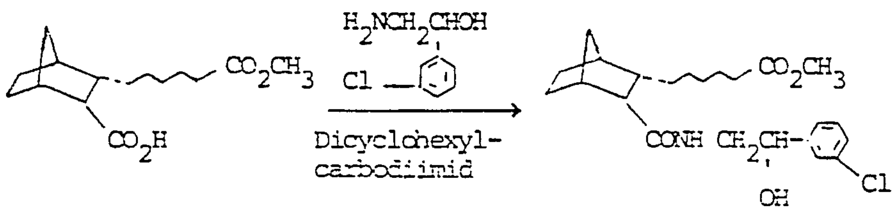


Die als Ausgangsstoffe verwendeten Lactone der allgemeinen Formel (II) sind bekannt.

Hexahydro-4,7-methano-iso-benzofuran-1(3H)-on ; Tetrahydro-4,7-methano-iso-benzofuran-1(3H)-on ; (J.C.S. Perkin I 1981, S. 3101, Tetrahedron Letters 1982, S. 539).

Entsprechend dem Reaktionsschema I werden in der ersten Stufe des Verfahrens die Lactone (II) mit Alkali-alkoxy-hydridoaluminaten, beispielsweise Natrium-bis-(2-methoxy-ethoxy)-di-hydrido-aluminat oder Natrium-ethoxy-bis-(2-methoxy-ethoxy)-hydrido-aluminat, oder mit Dialkylaluminiumhydriden, beispielsweise Diisobutylaluminiumhydrid, reduziert.

Ein besonders geeignetes Reduktionsmittel ist das Natrium-bis-(2-methoxy-ethoxy)-dihydrido-aluminat.

Als Verdünnungsmittel kommen inerte Kohlenwasserstoffe wie Toluol und Ether wie Tetrahydrofuran oder Diethylenglykoldimethylether infrage. Insbesondere eignet sich Toluol.

Die Reaktionstemperaturen liegen zwischen —78 und —40 °C, vorzugsweise zwischen —70 und —78 °C.

Die Reaktionszeit hängt von der Reaktionstemperatur ab und liegt zwischen 4 und 10 Stunden. Im allgemeinen setzt man 1 Mol des Lactons (II) mit mindestens einem Hydridäquivalent des Reduktionsmittels um. Ein Überschuß schadet nicht.

In einer zweiten Stufe des erfindungsgemäßen Verfahrens wird entsprechend dem Reaktionsschema I das Lactol der allgemeinen Formel (III) mit einem Phosphoniumsalz der allgemeinen Formel (IV) in Anwesenheit einer Base umgesetzt.

Die als Ausgangsstoffe verwendeten Phosphoniumsalze sind bekannt (J. Am. Chem. Soc. 1969, Bd. 91, S. 5675).

In der Formel (IV) steht n vorzugsweise für 2 bis 4, $R^7$ für Phenyl und X für Cl, Br, I, insbesondere für Br und Cl.

Beispielsweise seien folgende Verbindungen genannt :

3-Carboxy-propyl-triphenylphosphoniumbromid,
4-Carboxy-butyltriphenylphosphoniumbromid,
4-Carboxy-butyl-triphenylphosphoniumchlorid,
5-Carboxy-pentyl-triphenylphosphoniumbromid.

Als Base eignen sich Alkalihydride wie Natriumhydrid oder Kaliumhydrid, vorzugsweise Natriumhydrid.

Als Lösungsmittel kommt Dimethylsulfoxid infrage.

Die Reaktionstemperatur liegt zunächst in einem Bereich zwischen 30 und 90 °C, vorzugsweise von 60 bis 70 °C (Herstellung des Natriumsalzes des Dimethylsulfoxids) und anschließend in einem Temperaturbereich zwischen 10 und 40 °C, vorzugsweise 15 und 20 °C.

Die Reaktionszeit ist von der Temperatur abhängig und liegt im allgemeinen zwischen 1 und 6 Stunden.

Im allgemeinen setzt man 1 Mol des Lactols (III) mit 1,0 bis 3,0 Mol, vorzugsweise 2,0 bis 3,0 Mol eines Phosphoniumsalzes der allgemeinen Formel (IV) um, das zunächst mit 2,0 bis 6,0 Mol, vorzugsweise mit 4,0 bis 6,0 Mol Base umgesetzt wurde.

In einer dritten Stufe des Verfahrens wird entsprechend dem Reaktionsschema die Säure der allgemeinen Formel (V) mit einem Alkohol der allgemeinen Formel (VI) zur Reaktion gebracht. Als Alkohole eignen sich niedere Alkohole wie Methanol, Ethanol, Propanol, insbesondere Methanol und Ethanol.

Die Reaktion wird unter Zusatz einer zweiten Säure durchgeführt. Dafür eignen sich anorganische Säuren wie Salzsäure, Schwefelsäure, Phosphorsäure, vorzugsweise Schwefelsäure oder organische Säuren wie Methansulfonsäure, Benzolsulfonsäure, p-Toluolsulfonsäure, vorzugsweise p-Toluolsulfonsäure.

Als Verdünnungsmittel eignet sich ein Überschuß an Alkohol oder chlorierte Kohlenwasserstoffe wie Dichlormethan, Tetrachlorkohlenstoff, 1,2-Dichlorethan, Trichlorethylen, vorzugsweise Dichlormethan, Tetrachlorkohlenstoff oder die Kombination beider Verdünnungsmittel.

Die Reaktionstemperaturen können in einem größeren Bereich variiert werden. Im allgemeinen arbeitet man zwischen 60 und 97 °C, vorzugsweise unter Rückflußtemperatur.

Die Reaktionszeit ist von der Temperatur abhängig und liegt im allgemeinen zwischen 4 und 20

Stunden.

Im allgemeinen setzt man 1 Mol der Säure (V) mit 1,5 bis 6 Mol Alkohol (IV) unter Zusatz von 0,1 bis 0,2 Mol Säure um. Ein Überschuß an Alkohol schadet nicht.

Entsprechend dem Reaktionsschema I wird in einer vierten Stufe der erfindungsgemäßen Verfahrens eine Verbindung der allgemeinen Formel (VII) mit sechswertigem Chrom oxidiert.

Ein geeignetes Oxidationsmittel ist das Jones-Reagens, das heißt angesäuertes Chromtrioxid (J. Chem. Soc. 1947, S. 39). Als Verdünnungsmittel eignet sich Aceton.

Die Reaktionstemperatur liegt zwischen — 70 und + 20 °C, zweckmäßigerweise bei — 25 bis 0 °C. Die Reaktionszeit ist von der Temperatur abhängig und liegt im allgemeinen zwischen 2 und 4 Stunden.

Man setzt normalerweise 1 Mol des Alkohols (VII) mit der stöchiometrisch berechneten Menge, beziehungsweise mit einem bis 8-molaren Überschuß an Oxidationsmittel um.

Ein weiteres geeignets Oxidationsmittel ist das Pyridiniumdichromat (Tetrahedron Letters 1979, S. 399).

Als Verdünnungsmittel ist Dimethylformamid geeignet. Die Reaktionstemperatur liegt zwischen 0 und 40 °C, vorzugsweise zwischen 10 und 25 °C. Die Reaktionszeit ist von der Temperatur abhängig und liegt im allgemeinen zwischen 8 und 24 Stunden.

Man setzt im allgemeinen 1 Mol des Alkohols (VII) mit 0,5 bis 5 Mol, vorzugsweise mit 2 bis 4 Mol Pyridiniumdichromat um.

In der fünften Stufe des Verfahrens setzt man zuerst eine Säure der allgemeinen Formel (VIII) mit 1-Hydroxybenzotriazol unter Zusatz eines wasserentziehenden Agens um und dann mit einem Amin der allgemeinen Formel (IX) als Hydrochlorid unter Zugabe einer Base.

1-Hydroxybenzotriazol ist bekannt (J. prakt. Chem. Bd. 111, S. 272 (1925)).

Die Amine der allgemeinen Formel (IX) sind bekannt oder können nach bekannten Verfahren hergestellt werden (J. Org. Chem. Bd. 25, 257 (1960) ; J. Amer. Chem. Soc. Bd. 73, S. 2359 (1951) ; J. Org. Chem. Bd. 39, S. 914 (1974)).

In der Formel (IX) stehen vorzugsweise

$R^2$ für Wasserstoff oder Alkyl mit 1 bis 4 Kohlenstoffatomen, insbesondere für Wasserstoff und Methyl,

$R^3$ für Alkyl oder Alkenyl mit 1 bis 6 Kohlenstoffatomen, insbesondere für Propyl, Butyl, Pentyl oder für Cycloalkyl und Cycloalkenyl mit 5 bis 6 Kohlenstoffatomen, insbesondere für Cyclopentyl, Cyclohexyl, Cyclohexenyl oder für Phenyl und durch Halogen, vorzugsweise Fluor oder Chlor und durch Alkyl, vorzugsweise Methyl oder Trifluormethyl substituiertes Phenyl, insbesondere für Phenyl und durch Chlor oder Trifluormethyl substituiertes Phenyl, oder für Heteroaryl wie Pyridyl, Imidazolyl, Furyl, Thienyl, insbesondere für Pyridyl und Imidazolyl,

$R^4$ für Wasserstoff oder eine Hydroxygruppe,

$R^5$ und $R^6$ für Wasserstoff oder ein Alkyl mit 1-3 Kohlenstoffatomen, insbesondere für Wasserstoff.

Beispielsweise seien folgende Verbindungen genannt :

2-Hydroxyhexylamin,
2-Hydroxy-2-(cyclohex-3-en-yl)-ethylamin,
2-Hydroxy-2-methyl-pentylamin,
2-m-Chlorphenyl-2-hydroxy-ethylamin,
2-Hydroxy-2-phenyl-propylamin,
2-Phenyl-propylamin,
2-Methyl-pentylamin,
2-m-Chlorphenylethylamin,
2-m-Trifluormethylphenylethylamin.

Als Verdünnungsmittel kommen alle inerten organischen Lösungsmittel infrage. Hierzu gehören vorzugsweise Ether wie Tetrahydrofuran, Dioxan, Diethylenglykoldimethylether, Triethylenglykoldimethylether, insbesondere Tetrahydrofuran und Diethylenglykoldimethylether.

Als wasserentziehendes Angenzien eignen sich Carbodiimide, insbesondere Cyclohexylcarbodiimid.

Als Base verwendet man tertiäre Amine wie Triethylamin, N-Ethylmorpholin, Dimethylanilin, 1-Methylpiperidin, Dimethylcyclohexylamin, insbesondere Triethylamin und N-Ethylmorpholin.

Die Reaktionstemperaturen können in einem größeren Bereich variiert werden. Im allgemeinen arbeitet man zwischen — 10 und 40 °C, vorzugsweise zwischen — 5 und 30 °C.

Die Reaktionszeit ist von der Temperatur abhängig und liegt zwischen 2 und 6 Stunden.

Bei der Durchführung des erfindungsgemäßen Verfahrens setzt man 1 Mol der Säure (VIII) mit 1 Mol N-Hydroxybenzotriazol unter Zusatz von 1,0 bis 1,2 Mol, vorzugsweise 1,0 bis 1,1 Mol Cyclohexylcarbodiimid um und darauf mit 1,0 bis 1,1 Mol, insbesondere mit 1,0 bis 1,05 Mol Amin der allgemeinen Formel (IX).

In der sechsten Stufe des Verfahrens verseift man die Amid-Ester der allgemeinen Formel (I) zu den Amid-Säuren der allgemeinen Formel (I).

Als Verdünnungsmittel kommen wäßrige Alkohole wie Methanol, Ethanol oder Propanol, vorzugsweise Methanol infrage.

Als Basen können Alkalihydroxide, Alkalicarbonate verwendet werden. Als besonders geeignet seien im einzelnen genannt : Natriumhydroxid, Kaliumhydroxid.

Die Reaktionstemperaturen liegen zwischen 0 und 40 °C, vorzugsweise zwischen 10 und 30 °C.

Die Reaktionszeit hängt von der Reaktionstemperatur ab und liegt zwischen 12 und 48 Stunden.

Im allgemeinen wird 1 Mol des Amid-Esters (I) mit 1,0 bis 3,0 Mol, vorzugsweise mit 1,0 bis 1,5 Mol Base umgesetzt.

In einer variante des erfindungsgemäßen Verfahrens wird, wie im Reaktionsschema I beschrieben ist, der ungesättigte Ester (VII) hydriert.

Als Katalysatoren verwendet man Metallkatalysatoren, vorzugsweise Platin, Palladium, Raney-Nickel, insbesondere Palladium.

Die Wahl des Verdünnungsmittels hängt vom Katalysator ab. Setzt man Palladium auf Kohle ein, so eignen sich Alkohole wie Methanol, Ethanol, Propanol, insbesondere Methanol und Ethanol.

Die Reaktionstemperatur ist breit variierbar. Im allgemeinen arbeitet man bei 20 bis 100 °C, vorzugsweise bei 40 bis 70 °C.

Die Reaktionszeit ist von der Temperatur abhängig und liegt zwischen 1 und 4 Stunden.

Die Umsetzung wird bei erhöhtem Druck durchgeführt. Im allgemeinen arbeitet man bei Drücken zwischen 1,5 und 35 bar, vorzugsweise zwischen 10 und 30 bar.

Bei der Durchführung des erfindungsgemäßen Verfahrens setzt man 1 Mol des ungesättigten Esters (VII) mit einem Überschuß an Wasserstoff in Anwesenheit von 1 bis 100 mMol, vorzugsweise 1 bis 60 mMol Palladium auf Kohle um.

In einer weiteren Stufe des erfindungsgemäßen Verfahrens wird der Ester (X) mit sechswertigen Chrom zur Säure (XI) oxidiert, wie es in der vierten Stufe des Verfahrens beschrieben ist.

Analog der fünften und sechsten Stufe des erfindungsgemäßen Verfahrens wird darauf die Säure der allgemeinen Formel (XI) mit Aminen der allgemeinen Formel (IX) zu den Amid-Estern (I) beziehungsweise gegebenenfalls durch nachfolgende Verseifung zu den Amid-Säuren der allgemeinen Formel (I) umgesetzt.

Als für die erfindungsgemäßen Arzneimittel geeignet Wirkstoffe seien im einzelnen genannt :

6-[3-(N-2-Hydroxyhexyl)-carbamyl-bicyclo [2.2.1] hept-2-yl]-hex-5-en-säuremethylester
6-[3-(N-2-(Cyclohex-3-en-yl)-2-hydroxy-ethyl)-carbamyl-bicyclo [2.2.1] hept-2-yl]-hex-5-en-säuremethylester
6-[3-(N-2-Hydroxy-2-methyl-pentyl)-carbamyl-bicyclo [2.2.1] hept-2-yl]-hex-5-en-säuremethylester
6-[3-(N-2-m-Chlorphenyl-2-hydroxy-ethyl)-carbamyl-bicyclo [2.2.1] hept-2-yl]-hex-5-en-säuremethylester
6-[3-(N-2-Hydroxy-2-phenyl-propyl)-carbamyl-bicyclo-[2.2.1] hept-2-yl]-hex-5-en-säuremethylester
6-[3-(N-2-Phenyl-propyl)-carbamyl-bicyclo [2.2.1] hept-2-yl]-hex-5-en-säuremethylester
6-[3-(N-2-Methyl-pentyl)-carbamyl-bicyclo [2.2.1] hept-2-yl]-hex-5-en-säuremethylester
6-[3-(N-2-m-Chlorphenyl-ethyl)-carbamyl-bicyclo [2.2.1]-hept-2-yl]-hex-5-en-säuremethylester
6-[3-(N-2-Hydroxy-hexyl)-carbamyl-bicyclo [2.2.1] hept-5-en-2-yl]-hex-5-en-säuremethylester
6-[3-(N-2-Hydroxy-2-methyl-pentyl)-carbamyl-bicyclo [2.2.1]-hept-5-en-2-yl]-hex-5-en-säuremethylester
6-[3-(N-2-m-Chlorphenyl-2-hydroxy-ethyl)-carbamyl-bicyclo [2.2.1] hept-5-en-2-yl]-hex-5-en-säuremethylester
6-[3-(N-2-m-Trifluormethylphenyl-2-hydroxy-ethyl)-carbamyl-bicyclo [2.2.1] hept-5-en-2-yl]-hex-5-en-säuremethylester
6-[3-(N-2-Hydroxyhexyl)-carbamyl-bicyclo [2.2.1] hept-2-yl]-hex-5-en-säure
6-[3-(N-2-Hydroxy-2-(cyclohex-3-en-yl)-ethyl)-carbamyl-bicyclo[2.2.1]hept-2-yl]-hex-5-en-säure
6-[3-(N-2-Hydroxy-2-methyl-pentyl)-carbamyl-bicyclo[2.2.1]-hept-2-yl]-hex-5-en-säure
6-[3-(N-2-m-Chlorphenyl-2-hydroxy-ethyl)-carbamyl-bicyclo-[2.2.1]-hept-2-yl]-hex-5-en-säure
6-[3-(N-2-Hydroxy-2-phenyl-propyl)-carbamyl-bicyclo-[2.2.1]hept-2-yl]-hex-5-en-säure
6-[3-(N-2-Phenyl-propyl)-carbamyl-bicyclo[2.2.1]hept-2-yl]-hex-5-en-säure
6-[3-(N-2-Methyl-pentyl)-carbamyl-bicyclo[2.2.1]hept-2-yl]-hex-5-en-säure
6-[3-(N-2-m-Chlorphenyl-ethyl)-carbamyl-bicyclo[2.2.1]-hept-2-yl-hex-5-en-säure
6-[3-(N-2-Hydroxy-hexyl)-carbamyl-bicyclo[2.2.1]hept-5-en-2-yl]-hex-5-en-säure
6-[3-(N-2-Hydroxy-2-methyl-pentyl)-carbamyl-bicyclo-[2.2.1]hept-5-en-2-yl]-hex-5-en-säure
6-[3-(N-2-m-Chlorphenyl-2-hydroxy-ethyl)-carbamyl-bicyclo[2.2.1] hept-5-en-2-yl]-hex-5-en-säure
6-[3-(N-2-m-Trifluormethylphenyl-2-hydroxy-ethyl)-carbamyl-bicyclo[2.2.1]hept-5-en-2-yl]-hex-5-en-säure
6-[3-(N-2-Phenyl-propyl)-carbamyl-bicyclo[2.2.1]hept-2-yl]-hexan-säuremethylester
6-[3-(N-2-(Methyl-pentyl)-carbamyl-bicyclo[2.2.1]hept-2-yl]-hexan-säuremethylester
6-[3-(N-2-m-Chlorphenyl-ethyl)-carbamyl-bicyclo[2.2.1]-hept-2-yl]-hexansäuremethylester
6-[3-(N-2-Phenyl-propyl)-carbamyl-bicyclo[2.2.1]hept-2-yl]-hexansäure
6-[3-(N-2-Methyl-pentyl)-carbamyl-bicyclo[2.2.1]hept-2-yl]-hexansäure
6-[3-(N-2-m-Chlorphenyl-ethyl)-carbamyl-bicyclo[2.2.1]hept-2-yl]-hexansäure

Als Zubereitungsformen kommen die üblichen galenischen Applikationsformen in Frage, beispielsweise Cremes, Tabletten, Pillen, Kapseln, Suppositorien, Emulsionen, Infusions- und Injektionslösungen. Diese Zubereitungsformen werden nach an sich bekannten Methoden hergestellt unter Verwendung üblicher Hilfs- und Trägerstoffe.

Der Einsatz der so hergestellten Arzneimittel erfolgt je nach Bedarf z. B. durch lokale, parenterale oder orale Verabreichung.

Besonders geeignet sind Formulierungen, die die erfindungsgemäßen Verbindungen in Konzentrationen von etwa 0,1 bis 10 Gew.-% enthalten. Besonders bevorzugt sind wäßrige Lösungen, die gegebenenfalls auf einen pH-Wert von 6 bis 8 gepuffert sind.

Die Dosierung der Norbornan- und Norbornencarbonsäureamide in den erfindungsgemäßen Arzneimitteln liegt vorzugsweise in einem Bereich von 0,05 bis 100 mg/kg, insbesondere von 0,1 bis 20 mg/kg Körpergewicht.

Die in den erfindungsgemäßen Arzneimitteln enthaltenen Norbornan- und Norbornencarbonsäureamide sind als Thromboxanantagonisten und Plättchenaggregationshemmer zur Verhütung und Behandlung von thromboembolischen Erkrankungen im venösen und arteriellen Bereich, postoperativen Thrombosen und zur Förderung der Durchgängigkeit von chirurgisch verpflanzten Gefäßtransplantaten geeignet. Ferner eignen sich die neuen erfindungsgemäßen Verbindungen zur Verhütung und Behandlung von Arteriosklerose, ischämischen Erkrankungen, insbesondere von Herzinfarkten, transitorisch ischämischen Anfällen (TIA) und Schlaganfall, Angina pectoris, plötzlichem Herztod, peripheren Durchblutungsstörungen, Migräne und Erkrankungen der Atemwege wie Asthma, Bronchitis, Bronchiektasis, Pneumonie und Emphysem.

Methodik

Thrombozytenaggregationshemmung in vitro

Für die in vitro-Bestimmung der thrombozytenaggregationshemmenden Wirkung wird Blut von gesunden Spendern, die mindestens 14 Tage lang kein Medikament eingenommen hatten, verwendet. Das Blut wird in 3,8 %iger Natriumcitratlösung aufgenommen. Plättchenreiches Plasma (PRP) wird durch 20 Min. lange Zentrifugation bei 150 g bei Raumtemperatur gewonnen (Jürgens/Beller : Klinische Methoden der Blutgerinnungsanalyse ; Thieme Verlag, Stuttgart 1959). Die Plättchenaggregation wird nach der turbidometrischen Methode (Born, G.V.R. : J. Physiol. 162, 67, 1962) im Aggregometer bei 37 °C bestimmt. Hierzu wird PRP mit der Prüfsubstanz bei 37 °C inkubiert und anschließend die Aggregation durch Zugabe einer Kollagensuspension ausgelöst. Für die in-vitro-Versuche wird die minimal effektive Wirkstoffkonzentration (MEK) angegeben, die in den entsprechenden PRP-Proben die Thrombozytenaggregation hemmt.

Die aggregationshemmende Wirkung läßt sich auf eine direkte Wechselwirkung mit dem Thromboxan/$PGH_2$-Rezeptor zurückführen. Zur Bestimmung dieser spezifischen thromboxanantagonistischen Wirkung wird die Plättchenaggregation durch das stabile $PGH_2$-Analogon 9, 11-Epoxymethano $PGH_2$ (U 44 069), das als Thromboxan-Agonist wirkt, ausgelöst (Bundy, G. L. : Tetrahedron Lett. 1975, 1957-1960). Die Messung der Thrombozytenaggregation erfolgt analog dem Verfahren, welches für die kollageninduzierte Thrombozytenaggregation beschrieben ist.

Thrombozytenaggregationshemmung ex vivo

Für die ex vivo-Untersuchungen wurde den Tieren die Wirksubstanz in einer Tylosesuspension oral verabreicht. Nach 90 Minuten wurden die Tiere entblutet und das PRP mittels Zentrifugation gewonnen. Die Messung der Aggregationshemmung erfolgt analog dem Verfahren, welches für die in vitro-Versuche beschrieben ist, jedoch ohne Vorinkubation der Proben.

Ergebnisse

An den in der Tabelle 1 genannten Verbindungen wurde die Thrombozytenaggregationshemmung (in vitro) induziert durch Kollagen beziehungsweise U 44 069 und die Thrombozytenaggregationshemmung (ex vivo) bestimmt.

Die Norbornan- und Norbornencarbonsäureamide (siehe zum Beispiel Verbindung 20, 23, 24 oder 30) hemmen sowohl die Kollagen — als auch die U 44 069 — induzierte Thrombozytenaggregation stärker als AH 19 437, ein literaturbekannter Thromboxan-Antagonist, (Geisow, H. P., Hornby, E. J., McCabe, P. J., Brit. J. Pharmacol. 73, 219 P, 1981).

Die Norbornan- und Norbornencarbonsäureamide (siehe zum Beispiel Verbindung 23 oder 30) sind stärkere Hemmer der Kollagen-induzierten Aggregation als SQ 26 536 (Harris, D. N., Phillips, M. B., Mishel, I. M., Goldenberg H. J., Heikes, J. E., Sprague, P. W., Antonaccio, M. J., Prostaglandins 22, 295-307, 1981).

(Siehe Tabelle 1 Seite 10 f.)

Tabelle 1

| Verbindung gemäß Beispiel Nr. | Thrombozytenaggregationshemmung in vitro minimal effektive Konzentration (g/ml) | | Thrombozytenaggregationshemmung ex vivo | |
|---|---|---|---|---|
| | Kollagen-induziert | U 44069-induziert | Dosis (mg/kg) | Hemmung (90) |
| 10 | $1 \times 10^{-5} - 3 \times 10^{-6}$ | – | 100 | > 50 |
| 11 | $3 \times 10^{-6} - 1 \times 10^{-6}$ | $1 \times 10^{-5} - 3 \times 10^{-6}$ | | |
| 20 | $3 \times 10^{-6} - 1 \times 10^{-6}$ | $3 \times 10^{-6} - 1 \times 10^{-6}$ | 100 | > 50 |
| 21 | $3 \times 10^{-6} - 1 \times 10^{-6}$ | $1 \times 10^{-5} - 3 \times 10^{-6}$ | 100 | > 50 |
| | | | 30 | > 50 |
| | | | 10 | ~ 50 |
| 22 | $3 \times 10^{-6} - 1 \times 10^{-6}$ | $1 \times 10^{-5} - 3 \times 10^{-6}$ | 100 | ~ 50 |
| 23 | $3 \times 10^{-6} - 1 \times 10^{-6}$ | $1 \times 10^{-6} - 3 \times 10^{-7}$ | 100 | > 50 |
| 24 | $1 \times 10^{-6} - 3 \times 10^{-7}$ | $3 \times 10^{-6} - 1 \times 10^{-6}$ | 100 | > 50 |
| | | | 30 | < 50 |
| 29 | $1 \times 10^{-5} - 3 \times 10^{-6}$ | $1 \times 10^{-5} - 3 \times 10^{-6}$ | 100 | ~ 50 |
| 30 | $1 \times 10^{-6} - 3 \times 10^{-7}$ | $1 \times 10^{-6} - 3 \times 10^{-7}$ | 100 | > 50 |
| | | | 30 | > 50 |
| | | | 10 | < 50 |

Beispiel 1

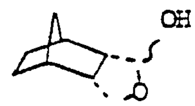

1-Hydroxy-octahydro-4,7-methano-iso-benzofuran

30,4 g (0,2 Mol) Hexahydro-4,7-methano-iso-benzofuran-1(3H)-on legt man unter Inertgas in 800 ml absolutem Toluol vor und läßt bei — 70 bis — 78 °C 84,8 ml (0,3 Mol) Natrium-bis-(2-methoxy-ethoxy)-di-hydridoaluminat (70 %ige Lösung in Toluol) verdünnt mit 200 ml absolutem Toluol einfließen. Man rührt 6 Stunden bei — 70 bis — 78 °C. Darauf tropft man 580 ml 50 % wäßriges Methanol bei — 70 °C zu, rührt 15 Minuten bei 20 °C nach und verdünnt mit 400 ml gesättigter wäßriger Natriumchlorid-Lösung. Die organische Phase wird abgetrennt und die wäßrige Phase mit Essigester extrahiert. Die organischen Phasen vereinigt man, wäscht sie mit gesättigter wäßriger Natriumchlorid-Lösung, trocknet über Natriumsulfat und dampft die Lösungsmittel im Vakuum ein. Man erhält auf diese Weise 30,1 g (98 % Ausbeute) 1-Hydroxy-octahydro-4,7-methano-iso-benzofuran.

$R_F$ = 0,38 [Methylenchlorid/Methanol (95 : 5)/Kieselgel]

Beispiel 2

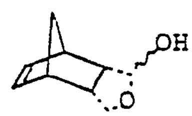

1-Hydroxy-hexahydro-4,7-methano-iso-benzofuran

Analog Beispiel 1 erhält man aus 30,0 g (0,2 Mol) Tetrahydro-4,7-methano-iso-benzo-furan-1(3H)-on 29,8 g (98 % Ausbeute) 1-Hydroxy-hexahydro-4,7-methano-iso-benzofuran.

$^{13}$C-NMR ($CDCl_3$) : δ = 100,278 ppm

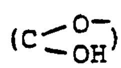

Beispiel 3

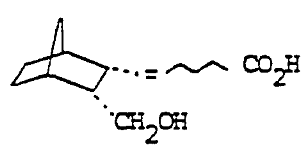

6-(3-Hydroxymethyl-bicyclo[2.2.1]hept-2-yl)-hex-5-en-säure

12 g (0,4 Mol) 80 %iges NaH gibt man unter Inertgas zu 240 ml absolutem Dimethylsulfoxid und erwärmt bis zum Ende der Wasserstoffentwicklung auf 60 bis 70 °C. Bei 15 bis 16 °C werden darauf 88,4 g (0,2 Mol) 4-Carboxy-butyl-triphenyl-phosphoniumbromid in 200 ml absolutem Dimethylsulfoxid zugegeben. Nach 10 Minuten Nachrühren läßt man 15,4 g (0,1 Mol) 1-Hydroxyoctahydro-4,7-methano-iso-benzofuran in 50 ml absolutem Dimethylsulfoxid einfließen und rührt 3 Stunden bei 20 °C. Man versetzt danach mit 20 ml Wasser, destilliert das Lösungsmittel im Vakuum ab und verteilt den Abdampfrückstand zwischen 200 ml Wasser und 4 mal 400 ml Diethylether. Die wäßrige Phase wird mit 5 n Salzsäure auf pH 3,5 eingestellt, der ausgefallene Niederschlag abgetrennt und 3 mal mit 200 ml Diethylether gewaschen. Die verbleibende wäßrige Phase wird mit Natriumchlorid gesättigt und 5 mal mit 200 ml Diethylether extrahiert. Nach Trocknung der vereinigten etherischen Extrakte dampft man das Lösungsmittel im Vakuum ab und chromatographiert den Rückstand an Kieselgel mit Methylenchlorid/Tetrahydrofuran/Essigsäure (10 : 1 : 0,5). Man erhält auf diese Weise 30,9 g 6-(3-Hydroxymethyl-bicyclo-[2.2.1]-hept-2-yl)-hex-5-en-säure (65 % Ausbeute).

$^1$H-NMR ($CDCl_3$) : δ = 5,00-5,39 ppm (Multiplett)

Beispiel 4

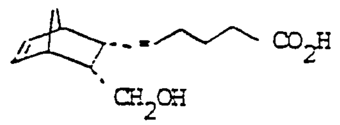

6-(3-Hydroxymethyl-bicyclo[2.2.1]hept-5-en-2-yl)-hex-5-en-säure

Analog Beispiel 3 erhält man 6-(3-Hydroxymethyl-bicyclo-[2.2.1]hept-5-en-2-yl)-hex-5-en-säure (Ausbeute : 66 %).

$^1$H-NMR ($CDCl_3$) : δ = 5,13-5,50 (Multiplett) und 5,89-6,27 (Multiplett) ppm

Beispiel 5

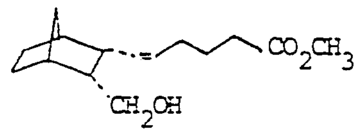

6-(3-Hydroxymethyl-bicyclo[2.2.1]hept-2-yl)-hex-5-en-säuremethylester

65,7 g (0,28 Mol) 6-(3-Hydroxymethyl-bicyclo[2.2.1]hept-2-yl)-hex-5-en-Säure erhitzt man in 220 ml Methanol und 600 ml Tetrachlorkohlenstoff unter Zusatz von 6,0 g (0,03 Mol) p-Toluolsulfonsäure 16 Stunden unter Rückfluß. Danach wäscht man 3 mal mit 500 ml gesättigter wäßriger Natriumhydrogencarbonat-Lösung und 2 mal mit 500 ml gesättigter wäßriger Natriumchlorid-Lösung. Man trocknet über Natriumsulfat und dampft dann die Lösungsmittel im Vakuum ab. Man erhält 66,4 g (Ausbeute : 95 %) 6-(3-Hydroxymethyl-bicyclo[2.2.1]hept-2-yl)-hex-5-en-säuremethylester.

$^1$H-NMR ($CDCl_3$) : δ = 3,62 ppm (Singulett)

Beispiel 6

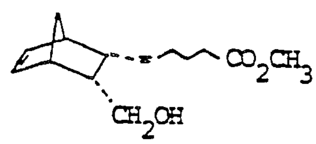

6-(3-Hydroxymethyl-bicyclo[2.2.1]hept-5-en-2-yl)-hex-5-en-säure-methylester

Man erhält analog Beispiel 56-(3-Hydroxymethyl-bicyclo-[2.2.1]hept-5-en-2-yl)-hex-5-en-säuremethylester (Ausbeute : 86 %).

1H-NMR($CDCl_3$) : δ = 5,95-6,32 (Multiplett), 5,17-5,47 (Multiplett) und 3,62 ppm (Singulett)

Beispiel 7

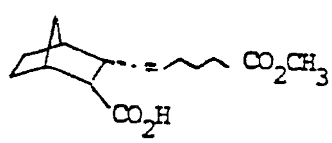

6-(3-Carboxy-bicyclo[2.2.1]hept-2-yl)-hex-5-en-säuremethylester

a) Zu 132,0 g (0,35 Mol) Pyridiniumdichromat in 200 ml absolutem Dimethylformamid gibt man bei 20 °C eine Lösung von 25,0 g (0,1 Mol) 6-(3-Hydroxymethyl-bicyclo[2.2.1]hept-2-yl)-hex-5-en-säuremethylester in 60 ml absolutem Dimethylformamid. Man rührt 9 Stunden bei 20 °C, läßt 14 Stunden stehen, gießt anschließend das Reaktionsgemisch in 1,9 l Wasser und extrahiert mit Diethylether. Nach der Trocknung über $Na_2SO_4$ dampft man im Vakuum ein, chromatographiert den Rückstand an Kieselgel mit Methylenchlorid/Methanol (95 : 5) und erhält 17,3 g 6-(3-Carboxy-bicyclo[2.2.1]hept-2-yl)-hex-5-en-säuremethylester (Ausbeute : 65 %)

$^1$H-NMR ($CDCl_3$) : δ = 10,9 (Singulett), 5,13-5,53 (Multiplett) und 3,70 ppm (Singulett)

b) In eine Lösung von 1,2 g (5 mMol 6-(3-Hydroxymethyl-bicyclo[2.2.1]t-2-yl)-hex-5-en-säuremethylester in 250 ml absolutem Aceton läßt man bei — 20 °C 14,5 ml Joneslösung (26,7 g Chromtrioxid, 23 ml conc. $H_2SO_4$, mit Wasser auf 100 ml) einfließen. Man rührt 3 Stunden bei — 20 °C gibt anschließend

40 ml 2-Propanol zu, darauf 30 ml Wasser und stellt den pH mit festem $NaHCO_3$ auf 4 ein. Man dekantiert vom Niederschlag, dampft die Lösung im Vakuum ein und extrahiert den Rückstand 3 mal mit Essigester. Nach Trocknung der Essigesterphase über Natriumsulfat dampft man im Vakuum ein und reinigt den 6-(3-Carboxy-bicyclo[2.21]hept-2-yl)-hex-5-en-säuremethylester wie unter a) beschrieben. 1,0 g (Ausbeute : 75 %).

Beispiel 8

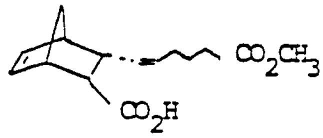

6-(3-Carboxy-bicyclo[2.2.1]hept-5-en-yl)-hex-5-en-säuremethylester

Wie in Beispiel 7a beschrieben führt die Oxidation von 6-(3-Hydroxymethyl-bicyclo[2.2.1]hept-5-en-2-yl)-hex-5-en-säuremethylester zu 6-(3-Carboxy-bicyclo[2.2.1]hept-5-en-2-yl)-hex-5-en-säuremethylester (Ausbeute : 59 %).

$^1$H-NMR ($CDCl_3$) : δ = 11,16 (Singulett), 5,93-6,40 (Multiplett), 5,22-5,55 (Multiplett) und 3,67 ppm (Singulett)

Beispiel 9

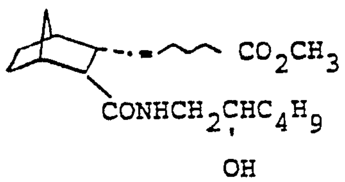

6-[3-(N-2-Hydroxy-hexyl)-carbamyl-bicyclo[2.2.1]hept-2-yl]-hex-5-en-säuremethylester

Zu einer Lösung von 2,66 g (10 Mmol) 6-(3-Carboxy-bicyclo[2.2.1]hept-2-yl)-hex-5-en-säuremethylester in 10 ml absolutem Tetrahydrofuran gibt man 1,35 g (10 mMol) N-Hydroxybenzotriazol und dann bei 0 °C 2,30 g (11 mMol) Dicyclohexylcarbodiimid. Es wird 1 Stunde bei 0 und 1 Stunde bei 20 °C gerührt. Darauf setzt man 1,54 g (10 mMol) 2-Hydroxyhexylamin-hydrochlorid und 1,00 g (10 mMol) Triethylamin in 3 ml absolutem Tetrahydrofuran zu und rührt 2 Stunden bei 20 °C. Der entstandene Niederschlag wird abgesaugt, mit Tetrahydrofuran gewaschen und die Tetrahydrofuran-Lösung im Vakuum eingedampft. Der Abdampfrückstand wird in 50 ml Essigester gelöst, die Lösung nacheinander mit 10 ml gesättigter wäßriger Natriumhydrogencarbonat-Lösung, 10 ml 2 n wäßriger Zitronensäure, 10 ml gesättigter wäßriger Natriumhydrogencarbonat-Lösung und 10 ml Wasser gewaschen und über Natriumsulfat getrocknet. Man dampft den Essigester im Vakuum ab, chromatographiert den Rückstand an Kieselgel mit Methylenchlorid/Methanol (95 : 5). Man erhält so 2,0 g (Ausbeute : 55 %) 6-(3-(N-2-Hydroxyhexyl)-carbamyl-bicyclo[2.2.1]hept-2-yl)-hex-5-en-säuremethylester.

IR (Film) : γ = 1 540, 1 650 und 1 740 $cm^{-1}$

Beispiele 10-19

Man setzt analog Beispiel 9 1 Mol einer Säure der allgemeinen Formel (VIII) mit 1 Mol Amin der allgemeinen Formel (IX) um und erhält die in Tabelle 2 aufgeführten Reaktionsprodukte.

(Siehe Tabelle 2 Seite 14 ff.)

Tabelle 2 : Hergestellte Verbindungen der allgemeinen Formel (I)

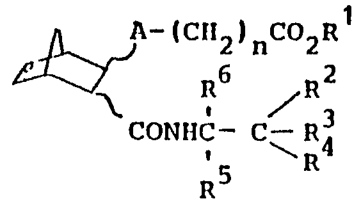 (I)

| Beispiel Nr. | $R^1$ | A | 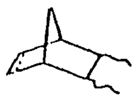 | $R^2$ | $R^3$ | $R^4$ | $R^5$ | $R^6$ | n | Ausbeute (%) | Physikalische Daten $^1H$-NMR ($CDCl_3$) (ppm), IR (Kap.) ($cm^{-1}$) |
|---|---|---|---|---|---|---|---|---|---|---|---|
| 10 | $CH_3$ | C=C | 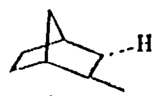 | | 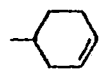 | OH | H | H | 3 | 66 | 6,50 (~ Triplett), 5,60-5,83 (Multiplett) |
| 11 | $CH_3$ | C=C | 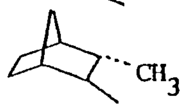 | | $C_3H_7$ | OH | H | H | 3 | 81 | 3,66 (Singulett), 1,13 (Singulett) |
| 12 | $CH_3$ | C=C | 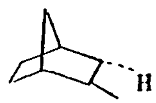 | | 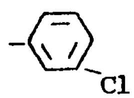 | OH | H | H | 3 | 56 | 1730, 1530, 1645 |
| 13 | $CH_3$ | C=C | 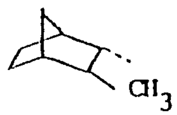 | | 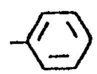 | OH | H | H | 3 | 77 | 3,97 (Dublett), 3,63 (Singulett) |
| 14 | $CH_3$ | C=C | 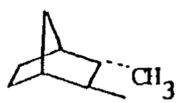 | | 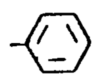 | H | H | H | 3 | 82 | 1730, 1640, 1530 |
| 15 | $CH_3$ | C=C | 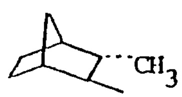 | | $C_3H_7$ | H | H | H | 3 | 76 | 5,63 (~Triplett), 3,63 Singulett) |

Tabelle 2 (Fortsetzung)

| Beispiel Nr. | $R^1$ | A 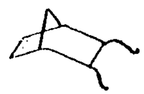 | $R^2$ | $R^3$ | $R^4$ | $R^5$ | $R^6$ | n | Ausbeute (%) | Physikalische Daten $^1H$-NMR ($CDCl_3$) (ppm), IR (Kap.) ($cm^{-1}$) |
|---|---|---|---|---|---|---|---|---|---|---|
| 16 | $CH_3$ | C=C 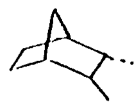 | H | 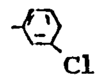 | H | H | H | 3 | 83 | 6,93-7,33 (Multiplett), 5,56 (~ Triplett) |
| 17 | $CH_3$ | C=C 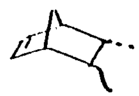 | H | $C_4H_9$ | OH | H | H | 3 | 77 | 1740, 1535, 1650 |
| 18 | $CH_3$ | C=C 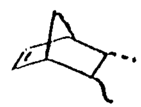 | $CH_3$ | $C_3H_7$ | OH | H | H | 3 | 64 | 5,96-6,47 (Multiplett) 5,23-5,53 (Multiplett) |
| 19 | $CH_3$ | C=C 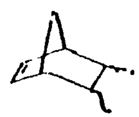 | H | 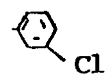 | OH | H | H | 3 | 81 | 3,60 (Singulett) |

Beispiel 20

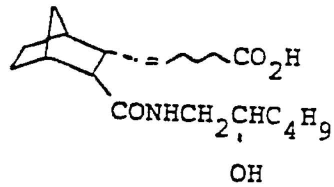

6-[3-(N-2-Hydroxy-hexyl)-carbamyl-bicyclo[2.2.1]hept-2-yl]-hex-5-en-säure

3,65 g (10 mMol) 6-[3(N-2-Hydroxyhexyl)-carbamyl-bicyclo[2.2.1]hept-2-yl]-hex-5-en-säuremethylester löst man in 50 ml Methanol und versetzt anschließend mit 10 ml 1 n NaOH und 6 ml Wasser. Man rührt 12 Stunden bei 20 °C, dampft nach weiteren 12 Stunden das Reaktionsgemisch im Vakuum ein, nimmt den Abdampfrückstand in 100 ml Wasser auf, säuert mit 2 n HCl auf pH 1 an und extrahiert 5 mal mit 50 ml Diethylether. Nach Trocknung der organischen Phase über Natriumsulfat dampft man diese im Vakuum ein und chromatographiert falls erforderlich an Kieselgel mit Methylenchlorid/Methanol (9 : 1). Man erhält 2,46 g 6-[3(N-2-Hydroxy-hexyl)-carbamyl-bicyclo [2.2.1]hept-2-yl]-hex-5-en-säure (Ausbeute : 70 %).

$^1$H-NMR($CDCl_3$) : $\delta$ = 6,25 (~ Triplett) und 0,88 ppm (~ Triplett)

Beispiele 21-30

Analog Beispiel 20 verseift man die Amid-Ester der allgemeinen Formel (I) mit der äquivalenten Menge 1 n Natronlauge und erhält die in Tabelle 3 aufgeführten Amid-Säuren (I).

Einige Amid-säuren (I) fallen beim Ansäuern bereits aus und können durch Filtration isoliert werden.

(Siehe Tabelle 3 Seite 17 ff.)

Tabelle 3 : Hergestellte Verbindungen der allgemeinen Formel (I)

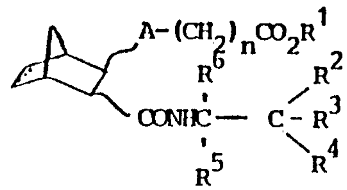

(I)

| Beispiel Nr. | $R^1$ | A | 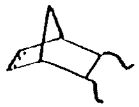 | $R^2$ | $R^3$ | $R^4$ | $R^5$ | $R^6$ | n | Ausbeute (%) | Physikalische Daten $^1H$-NMR ($CDCl_3$) (ppm), IR (Kap. o. KBr) ($cm^{-1}$) |
|---|---|---|---|---|---|---|---|---|---|---|---|
| 21 | H | C=C | 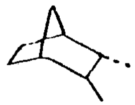 | H | 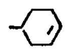 | OH | H | H | 3 | 73 | 5,53-5,87 (Multiplett), 5,00-5,43 (Multiplett) |
| 22 | H | C=C | 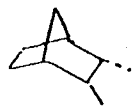 | $CH_3$ | $C_3H_7$ | OH | H | H | 3 | 73 | 1710, 1650, 1560 |
| 23 | H | C=C | 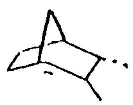 | H | 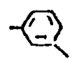 Cl | OH | H | H | 3 | 89 | 6,10 (~Triplett), 4,67-4,90 (Multiplett) |
| 24 | H | C=C | 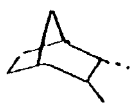 | $CH_3$ | 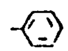 | OH | H | H | 3 | 79 | 5,08-5,32 (Multiplett), 1,50 (Singulett) |
| 25 | H | C=C | 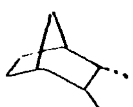 | $CH_3$ | 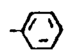 | H | H | H | 3 | 85 | 5,60 (~Triplett) |
| 26 | H | C=C | 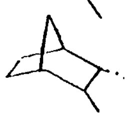 | $CH_3$ | $C_3H_7$ | H | H | H | 3 | 91 | 5,71 (~Triplett), 9,47 (Singulett) |

Tabelle 3 (Fortsetzung)

| Beispiel Nr. | $R^1$ | A | 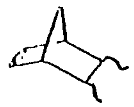 | $R^2$ | $R^3$ | $R^4$ | $R^5$ | $R^6$ | n | Ausbeute (%) | Physikalische Daten $^1H$-NMR($CDCl_3$) (ppm), IR (Kap. o. KBr) ($cm^{-1}$) |
|---|---|---|---|---|---|---|---|---|---|---|---|
| 27 | H | C=C | 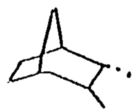 | H | 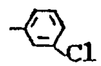 | H | H | H | 3 | 88 | 7,02-7,25 (Multiplett), 5,62 (~ Triplett) |
| 28 | H | C=C | 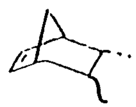 | H | $C_4H_9$ | OH | H | H | 3 | 71 | 1710, 1650, 1540 |
| 29 | H | C=C | 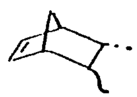 | $CH_3$ | $C_3H_7$ | OH | H | H | 3 | 76 | 1,10 (Singulett) |
| 30 | H | C=C | 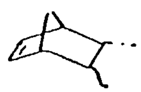 | H | 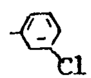 | OH | H | H | 3 | 88 | 5,83-6,57 (Multiplett) 5,17-5,57 (Multiplett) |

Beispiel 31

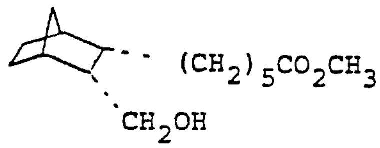

6-(3-Hydroxymethyl-bicyclo[2.2.1]hept-2-yl)-hexansäuremethylester

a) 28,7 g (0,11 Mol) 6-(3-Hydroxymethyl-bicyclo[2.2.1]-hept-2-yl)-hex-2-ensäuremethylester werden in 450 ml Methanol unter Zusatz von 12 g Palladium/Kohle (5 %ig) 1 1/2 Stunden bei 60 °C und unter einem Druck von 30 bar hydriert. Man entfernt den Katalysator durch Filtration, dampft das Methanol im Vakuum ab, chromatographiert das zurückbleibende Öl an Kieselgel mit Methylenchlorid/Methanol (99 : 1) und erhält 25,5 g (91 % Ausbeute) 6-(3-Hydroxymethyl-bicyclo[2.2.1]hept-2-yl)-hexansäuremethylester.

$^1$H-NMR ($CDCl_3$) : $\delta$ = 3,63 ppm (Singulett)

b) Analog Beispiel 31 a erhält man aus 6-(3-Hydroxymethylbicyclo[2.2.1]hept-5-en-2-yl)-hex-2-ensäuremethylester 6-(3-Hydroxymethyl-bicyclo[2.2.1]hept-2-yl)-hexansäuremethylester in 77 % Ausbeute.

Beispiel 32

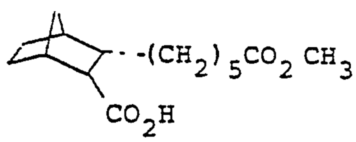

6-(3-Carboxy-bicyclo[2.2.1]hept-2-yl)-hexansäuremethylester

Gemäß dem in Beispiel 7a beschriebenen Verfahren erhält man aus 6-(3-Hydroxymethyl-bicyclo[2.2.1]hept-2-yl)-hexansäuremethylester den 6-(3-Carboxy-bicyclo[2.2.1]-hexansäuremethylester in 61 % Ausbeute. IR (Film) : $\gamma$ = 1 710 und 1 730 $cm^{-1}$.

Beispiele 33-38

Wie in Beispiel 9 beziehungsweise Beispiel 20 beschrieben, erhält man die in Tabelle 4 aufgeführten Amid-Ester und Amid-Säuren der allgemeinen Formel (I).

(Siehe Tabelle 4 Seite 20 f.)

Tabelle 4 : Hergestellte Verbindungen der allgemeinen Formel (I)

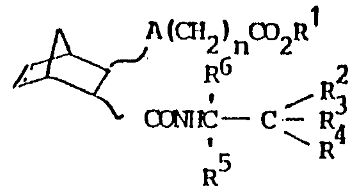

| Beispiel Nr. | $R^1$ | n | A | 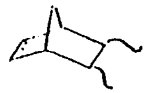 | $R^2$ | $R^3$ | $R^4$ | $R^5$ | $R^6$ | Ausbeute (%) | Physikalische Daten $^1H$-NMR ($CDCl_3$) (ppm), IR (Kap.) ($cm^{-1}$) |
|---|---|---|---|---|---|---|---|---|---|---|---|
| 33 | $CH_3$ | 3 | $CH_2CH_2$ | 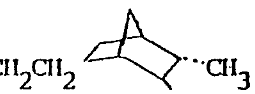 | $CH_3$ | 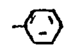 | H | H | H | 82 | 5,45 (~Triplett), 3,65 (Singulett) |
| 34 | $CH_3$ | 3 | $CH_2CH_2$ | 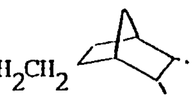 | $CH_3$ | $C_3H_7$ | H | H | H | 76 | 1730, 1640, 1530 |
| 35 | $CH_3$ | 3 | $CH_2CH_2$ | 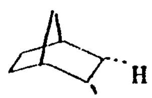 | H | 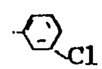 | H | H | H | 83 | 6,97-7,33 (Multiplett), 3,63 (Singulett) |
| 36 | H | 3 | $CH_2CH_2$ | 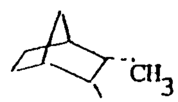 | $CH_3$ | 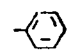 | H | H | H | 85 | 1710, 1640, 1530 |
| 37 | H | 3 | $CH_2CH_2$ | 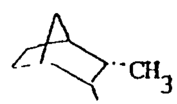 | $CH_3$ | $C_3H_7$ | H | H | H | 91 | 9,50 (Singulett) 5,71 (~Triplett) |
| 38 | H | 3 | $CH_2CH_2$ | 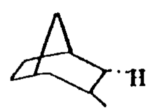 | H | 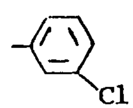 | H | H | H | 88 | 8,11 (Singulett), 5,62 (~Triplett) |

**Patentansprüche**

1. Norbornan- und Norbornencarbonsäureamide der allgemeinen Formel

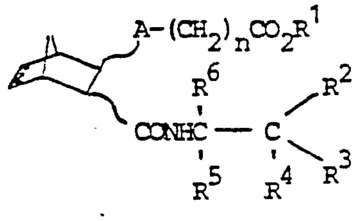

in welcher

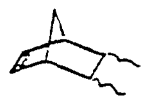

für die Teilstrukturen steht,

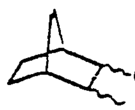
oder
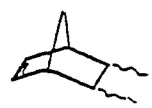

A für eine

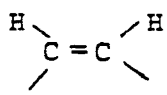

oder $—CH_2CH_2$-Gruppe steht,

$R^1$ und $R^2$ für Wasserstoff oder einen Alkylrest mit 1 bis 6, insbesondere 1-2 Kohlenstoffatomen stehen,

$R^3$ für ein gegebenenfalls durch Halogen oder Alkyl mit 1 bis 2 Kohlenstoffatomen substituiertes Alkyl oder Alkenyl mit 1-8 Kohlenstoffatomen oder für ein Cycloalkyl und Cycloalkenyl mit 3 bis 7 Kohlenstoffatomen oder einen gegebenenfalls durch Halogen, Alkyl mit 1 bis 2 Kohlenstoffatomen oder Halogenalkyl mit 1 bis 2 Kohlenstoffatomen substituierten Aromaten mit 6 oder 10 Kohlenstoffatomen oder für einen fünf- oder sechsgliedrigen Heteroaromaten steht,

$R^4$ für Wasserstoff oder eine Hydroxygruppe steht,

$R^5$ und $R^6$ gleich oder verschieden sind und für Wasserstoff oder Alkyl mit 1 bis 4 Kohlenstoffatomen stehen,

n die Zahlen 2-6, insbesondere 2-4 bedeuten, und, falls $R^1$ Wasserstoff bedeutet, auch deren physiologisch vertretbare Salze.

2. Verbindungen nach Anspruch 1, in denen $R^3$ für Propyl, Butyl, Pentyl, Cyclopentyl, Cyclohexyl, Cyclohexenyl, Phenyl, durch Chlor oder Trifluormethyl substituiertes Phenyl, Pyridyl oder Imidazolyl steht.

3. 6-[3-(N-2-Hydroxy-hexyl)-carbamyl-bicyclo[2.2.1]-hept-2-yl]-hex-5-en-säure.

4. 6-[3-(N-2-m-Chlorphenyl-2-hydroxy-ethyl)-carbamyl-bicyclo[2.2.1]-hept-2-yl]-hex-5-en-säure.

5. 6-[3-(N-2-Hydroxy-2-phenyl-propyl)-carbamyl-bicyclo[2.2.1]hept-2-yl]-hex-5-en-säure.

6. 6-[3-(N-2-m-Chlorphenyl-2-hydroxy-ethyl)-carbamyl-bicyclo[2.2.1]-hept-5-en-2-yl]-hex-5-en-säure.

7. Norbornan- und Norbornencarbonsäureamide der allgemeinen Formel

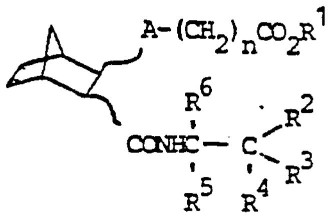

in welcher

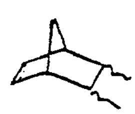

für die Teilstrukturen

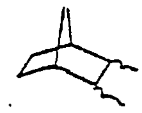 oder 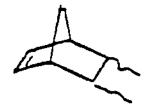

steht,

A für eine

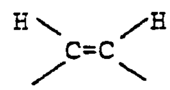

oder $-CH_2CH_2$-Gruppe steht,

$R^1$ und $R^2$ für Wasserstoff oder einen Alkylrest mit 1 bis 6, insbesondere 1-2 Kohlenstoffatomen stehen,

$R^3$ für ein gegebenenfalls durch Halogen oder Alkyl mit 1 bis 2 Kohlenstoffatomen substituiertes Alkyl oder Alkenyl mit 1-8 Kohlenstoffatomen oder für ein Cycloalkyl und Cycloalkenyl mit 3 bis 7 Kohlenstoffatomen oder einen gegebenenfalls durch Halogen, Alkyl mit 1 bis 2 Kohlenstoffatomen oder Halogenalkyl mit 1 bis 2 Kohlenstoffatomen substituierten Aromaten mit 6 oder 10 Kohlenstoffatomen oder für einen fünf- oder sechsgliedrigen Heteroaromaten steht,

$R^4$ für Wasserstoff oder eine Hydroxygruppe steht,

$R^5$ und $R^6$ gleich oder verschieden sind und für Wasserstoff oder Alkyl mit 1 bis 4 Kohlenstoffatomen stehen,

n die Zahlen 2-6, insbesondere 2-4 bedeuten, und, falls $R^1$ Wasserstoff bedeutet, auch deren physiologisch vertretbaren Salze, zur Verhütung und Behandlung von unter Thromboxanbeteiligung ablaufenden Erkrankungen.

8. Verfahren zur Herstellung von Norbornan- und Norbornencarbonsäureamide der allgemeinen Formel

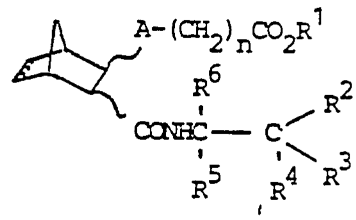

in welcher

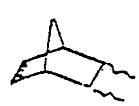

für die Teilstrukturen

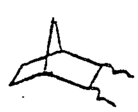 oder 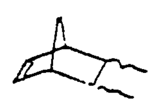

steht,

A für eine

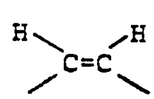

oder $-CH_2CH_2$-Gruppe steht,

$R^1$ und $R^2$ für Wasserstoff oder einen Alkylrest mit 1 bis 6, insbesondere 1-2 Kohlenstoffatomen

stehen,

$R^3$ für ein gegebenenfalls durch Halogen oder Alkyl mit 1 bis 2 Kohlenstoffatomen substituiertes Alkyl oder Alkenyl mit 1-8 Kohlenstoffatomen oder für ein Cycloalkyl und Cycloalkenyl mit 3 bis 7 Kohlenstoffatomen oder einen gegebenenfalls durch Halogen, Alkyl mit 1 bis 2 Kohlenstoffatomen oder Halogenalkyl mit 1 bis 2 Kohlenstoffatomen substituierten Aromaten mit 6 oder 10 Kohlenstoffatomen oder für einen fünf- oder sechsgliedrigen Heteroaromaten steht,

$R^4$ für Wasserstoff oder eine Hydroxygruppe steht,

$R^5$ und $R^6$ gleich oder verschieden sind und für Wasserstoff oder Alkyl mit 1 bis 4 Kohlenstoffatomen stehen,

n die Zahlen 2-6, insbesondere 2-4 bedeuten, und, falls $R^1$ Wasserstoff bedeutet, auch deren physiologisch vertretbaren Salze,

dadurch gekennzeichnet, daß man ein Lacton der allgemeinen Formel (II)

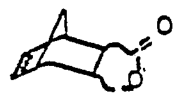

(II)

in welcher

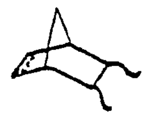

für die Teilstrukturen

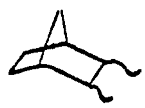

oder

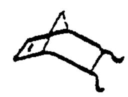

steht, zu einem Lactol der allgemeinen Formel (III)

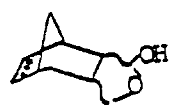

in welcher

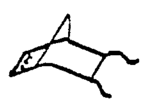

die obige Bedeutung hat, reduziert und das so erhaltene Lactol mit einem Phosphoniumsalz der allgemeinen Formel (IV)

$$[(R^7)_3\overset{+}{P}(CH_2)_{n+1}\ CO_2H]X^- \qquad (IV)$$

in welcher

$R^7$ für einen Arylrest

X für ein Halogen und

n für eine Zahl zwischen 2 und 6 steht,

in Anwesenheit einer Base zu einer Säure der allgemeinen Formel (V)

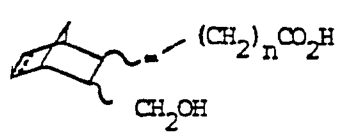

(V)

in welcher

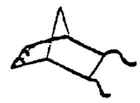

und n die obige Bedeutung haben, umsetzt und anschließend diese mit einem Alkohol der allgemeinen Formel (VI)

$R^8OH$ (VI)

in welcher $R^8$ für einen Alkylrest mit 1 bis 6 Kohlenstoffatomen steht, zu dem Ester der allgemeinen Formel (VII)

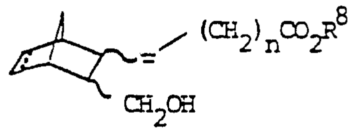

(VII)

in welcher

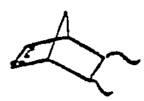

n und $R^8$ die oben angegebene Bedeutung haben, umsetzt, und, falls in der allgemeinen Formel (I) A für

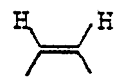

steht, diese zu einer Säure der allgemeinen Formel (VIII)

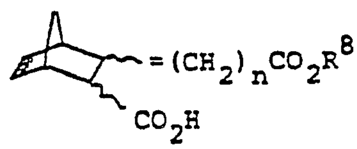

(VIII)

in welcher

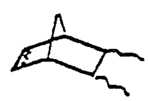

n und $R^8$ die obige Bedeutung haben, oxidiert und die so erhaltene Säure unter Überführung in einen aktivierten Ester mit einem Amin der allgemeinen Formel (IX)

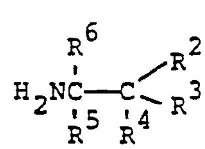

(IX)

in welcher

$R^2$ für Wasserstoff, oder einen Alkylrest mit 1-6 Kohlenstoffatomen steht,

$R^3$ für einen gegebenenfalls durch Halogen oder Alkyl mit 1 bis 2 Kohlenstoffatomen substituiertes Alkyl oder Alkenyl mit 1-8 Kohlenstoffatomen oder für ein Cycloalkyl und Cycloalkenyl mit 3 bis 7 Kohlenstoffatomen oder einen gegebenenfalls durch Halogen, Alkyl, mit 1 bis 2 Kohlenstoffatomen oder Halogenalkyl mit 1 bis 2 Kohlenstoffatomen substituierten Aromaten mit 6 oder 10 Kohlenstoffatomen oder für einen fünf- oder sechsgliedrigen Heteroaromaten steht,

$R^4$ für Wasserstoff oder eine Hydroxygruppe steht,

$R^5$ und $R^6$ gleich oder verschieden sind und für Wasserstoff oder Alkyl mit 1 bis 4 Kohlenstoffatomen stehen,

zu den Amid-Estern (I) oder unter folgender Verseifung, zu den Amid-Säuren (I) umsetzt, oder falls in der

allgemeinen Formel (I) A für eine $-CH_2CH_2$-Gruppe und

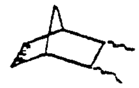

für die Teilstruktur

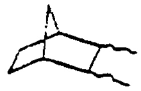

steht, den ungesättigten Ester der allgemeinen Formel (VII) zu dem Ester der allgemeinen Formel (X)

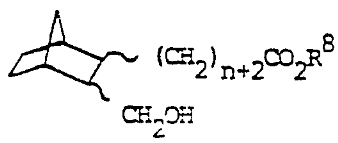
(X)

in welcher $R^8$ und n die obengenannte Bedeutung haben, hydriert, diesen zu einer Säure der allgemeinen Formel (XI)

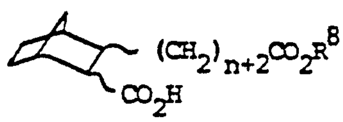
(XI)

in welcher $R^8$ und n die obige Bedeutung besitzen, umsetzt und die erhaltene Säure (XI) gegebenenfalls unter Überführung in einen aktivierten Ester mit einem Amin der allgemeinen Formel (IX) zu den Amid-Estern (I) oder unter folgender Verseifung zu den Amid-Säuren (I) umsetzt.

## Claims

1. Norbornane- and norbornene-carboxylic acid amides of the general formula

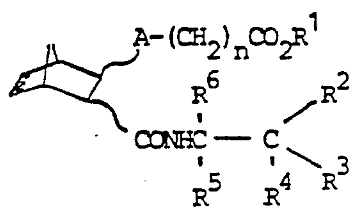

in which

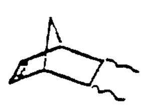

represents the part structures

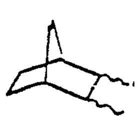 or 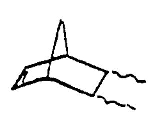

A represents a

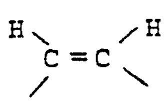

or $-CH_2CH_2-$ group,

$R^1$ and $R^2$ represent hydrogen or an alkyl radical with 1 to 6, in particular 1-2 carbon atoms,

$R^3$ represents alkyl or alkenyl which has 1-8 carbon atoms and is optionally substituted by halogen or alkyl with 1 to 2 carbon atoms, or represents cycloalkyl and cycloalkenyl with 3 to 7 carbon atoms, or an aromatic radical which has 6 or 10 carbon atoms and is optionally substituted by halogen, alkyl with 1 to 2 carbon atoms or halogenoalkyl with 1 to 2 carbon atoms, or represents à five-membered or six-membered heteroaromatic radical,

$R^4$ represents hydrogen or a hydroxyl group,

$R^5$ and $R^6$ are identical or different and represent hydrogen or alkyl with 1 to 4 carbon åtoms and n denotes the numbers 2-6, in particular and, if $R^1$ denotes hydrogen, also physiologically acceptable salts thereof.

2. Compounds according to Claim 1, in which $R^3$ represents propyl, butyl, pentyl, cyclopentyl, cyclohexyl, cyclohexenyl, phenyl, phenyl which is substituted by chlorine or trifluoromethyl, pyridyl or imidazolyl.

3. 6-[3-(N-2-Hydroxy-hexyl)-carbamyl-bicyclo[2.2.1]-hept-2-yl]-hex-5-enoic acid.

4. 6-[3-(N-3-m-Chlorophenyl-2-hydroxy-ethyl)-carbamyl-bicyclo[2.2.1]-hept-2-yl]-hex-5-enoic acid.

5. 6-[3-(N-2-Hydroxy-2-phenyl-propyl)-carbamyl-bicyclo[2.2.1]hept-2-yl]-hex-5-enoic acid.

6. 6-[3-(N-2-m-Chlorophenyl-2-hydroxy-ethyl)-carbamyl-bicyclo[2.2.1]-hept-5-en-2-yl]-hex-5-enoic acid.

7. Norbornane- and norbornene-carboxylic acid amides of the general formula

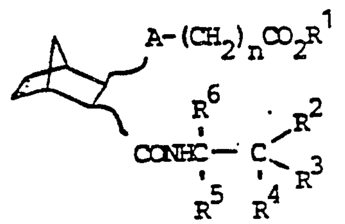

in which

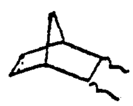

represents the part structures

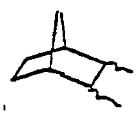 or 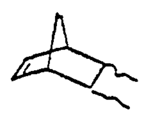

A represents a

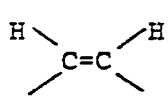

or $-CH_2CH_2-$ group,

$R^1$ and $R^2$ represent hydrogen or an alkyl radical with 1 to 6, in particular 1-2, carbon atoms,

$R^3$ represents alkyl or alkenyl which has 1-8 carbon atoms and is optionally substituted by halogen or alkyl with 1 to 2 carbon atoms, or represents cycloalkyl and cycloalkenyl with 3 to 7 carbon atoms, or an aromatic radical which has 6 or 10 carbon atoms and is optionally substituted by halogen, alkyl with 1 to 2 carbon atoms or halogenoalkyl with 1 to 2 carbon atoms, or represents a five-membered or six-membered heteroaromatic radical,

$R^4$ represents hydrogen or a hydroxyl group, $R^5$ and $R^6$ are identical or different and represent hydrogen or alkyl with 1 to 4 carbon atoms and

n denotes the numbers 2-6, in particular 2-4, and, if $R^1$ denotes hydrogen, also physiologically acceptable salts thereof, for preventing and treating diseases which proceed with participation of thromboxane.

8. Process for the preparation of norbornane- and norbornene-carboxylic acid amides of the general

formula

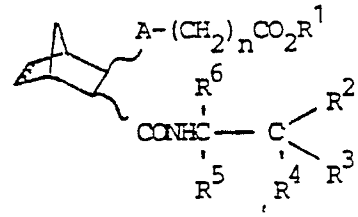

in which

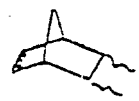

represents the part structures

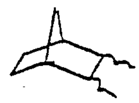

or

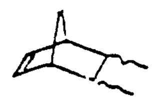

A represents a

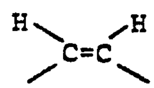

or $—CH_2CH_2—$ group,

$R^1$ and $R^2$ represent hydrogen or an alkyl radical with 1 to 6, in particular 1-2 carbon atoms,

$R^3$ represents alkyl or alkenyl which has 1-8 carbon atoms and is optionally substituted by halogen or alkyl with 1 to 2 carbon atoms, or represents cycloalkyl and cycloalkenyl with 3 to 7 carbon atoms, or an aromatic radical which has 6 or 10 carbon atoms and is optionally substituted by halogen, alkyl with 1 to 2 carbon atoms or halogenoalkyl with 1 to 2 carbon atoms, or represents a five-membered or six-membered heteroaromatic radical,

$R^4$ represents hydrogen or a hydroxyl group,

$R^5$ and $R^6$ are identical or different and represent hydrogen or alkyl with 1 to 4 carbon atoms and

n denotes the numbers 2-6, in particular 2-4,

and, if $R^1$ denotes hydrogen, also physiologically acceptable salts thereof, characterised in that a lactone of the general formula (II)

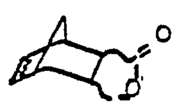

in which

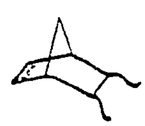

represents the part structures

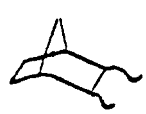

or

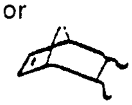

is reduced to give a lactol of the general formula (III)

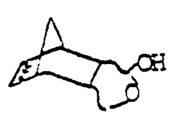

in which

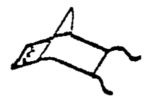

has the above meaning, and the lactol thus obtained is reacted with a phosphonium salt of the general formula (IV)

$$[(R^7)_3\overset{+}{P}(CH_2)_{n+1}\ CO_2H]X^- \qquad (IV)$$

in which
R⁷ represents an aryl radical,
X represents a halogen and
n represents a number between 2 and 6,
in the presence of a base, to give an acid of the general formula (V)

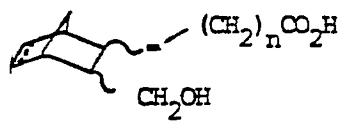

(V)

in which

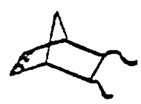

and n have the above meaning, and this acid is then reacted with an alcohol of the general formula (VI)

$R^8OH$ (VI)

in which $R^8$ represents an alkyl radical with 1 to 6 carbon atoms, to give the ester of the general formula (VII)

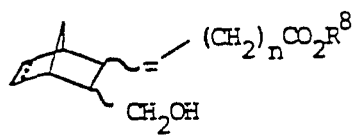

(VII)

in which

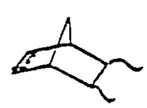

n and $R^8$ have the abovementioned meaning, and, if A in the general formula (I) represents

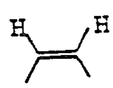

this ester is oxidised to give an acid of the general formula (VIII)

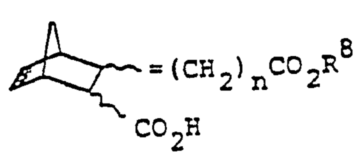

(VIII)

in which

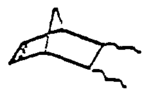

n and $R^8$ have the above meaning, and the acid thus obtained is reacted, with conversion into an activated ester, with an amine of the general formula (IX)

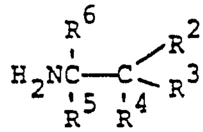

(IX)

in which

$R^2$ represents hydrogen or an alkyl radical with 1-6 carbon atoms,

$R^3$ represents alkyl or alkenyl which has 1-8 carbon atoms and is optionally substituted by halogen or alkyl with 1 to 2 carbon atoms, or represents cycloalkyl and cycloalkenyl with 3 to 7 carbon atoms, or an aromatic radical which has 6 or 10 carbon atoms and is optionally substituted.

**Revendications**

1. Amides d'acides norbornane-carboxyliques et norbornène-carboxyliques, de formule générale :

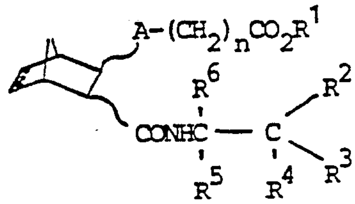

dans laquelle

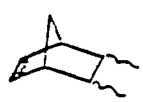

représente les structures partielles

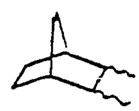

ou

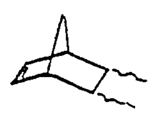

A représente un groupe

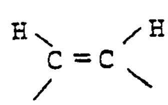

ou $—CH_2CH_2—$.

$R^1$ et $R^2$ représentent chacun un atome d'hydrogène ou un reste alkyle ayant 1 à 6, en particulier 1 ou

2, atomes de carbone,

$R^3$ représente un groupe alkyle ou alcényle ayant 1 à 8 atomes de carbone, éventuellement substitué par de l'halogène ou par un groupe alkyle ayant 1 à 2 atomes de carbone, ou il représente un groupe cycloalkyle et cycloalcényle ayant 3 à 7 atomes de carbone ou un groupe aromatique ayant 6 ou 10 atomes de carbone, éventuellement substitué par de l'halogène, par un groupe alkyle ayant 1 à 2 atomes de carbone ou halogénoalkyle ayant 1 à 2 atomes de carbone, ou bien il représente un groupe hétéroaromatique ayant 5 ou 6 chaînons,

$R^4$ représente un atome d'hydrogène ou un groupe hydroxy,

$R^5$ et $R^6$ sont identiques ou différents et représentent chacun un atome d'hydrogène ou un groupe alkyle ayant 1 à 4 atomes de carbone,

n représente des nombres valant 2 à 6, en particulier 2 à 4, et si $R^1$ représente un atome d'hydrogène, leurs sels physiologiquement compatibles.

2. Composés selon la revendication 1, dans lesquels $R^3$ représente un groupe propyle, butyle, pentyle, cyclopentyle, cyclohexyle, cyclohexényle, phényle, phényle (substitué par un atome de chlore ou par un groupe trifluorométhyle), pyridyle ou imidazolyle.

3. Acide 6-[3-(N-2-hydroxy-hexyl)-carbamyl-bicyclo-[2.2.1]hept-2-yl]-hex-5-ène-oïque.

4. Acide 6-[3-(N-2-m-chlorophényl-2-hydroxy-éthyl)-carbamyl-bicyclo [2.2.1] hept-2-yl]-hex-5-ène-oïque.

5. Acide 6-[3-(N-2-hydroxy-2-phényl-propyl)-carbamyl-bicyclo [2.2.1] hept-2-yl]-hex-5-ène-oïque.

6. Acide 6-[3-(N-2-m-chlorophényl-2-hydroxy-éthyl)-carbamyl-bicyclo [2.2.1] hept-5-ène-2-yl]-hex-5-ène-oïque.

7. Amides d'acides norbornane-carboxyliques et norbornène-carboxyliques, de formule générale :

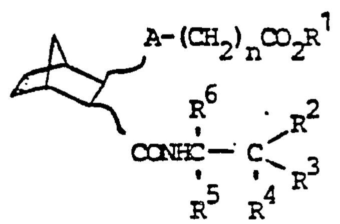

dans laquelle

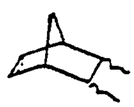

représente les structures partielles

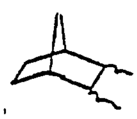 ou 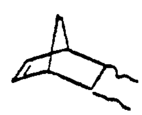

A représente un groupe

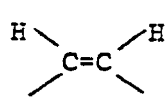

ou $-CH_2CH_2-$,

$R^1$ et $R^2$ représentent chacun un atome d'hydrogène ou un reste alkyle ayant 1 à 6, en particulier 1 ou 2, atomes de carbone,

$R^3$ représente un groupe alkyle ou alcényle ayant 1 à 8 atomes de carbone, éventuellement substitué par de l'halogène ou par un groupe alkyle ayant 1 à 2 atomes de carbone, ou il représente un groupe cycloalkyle et cycloalcényle ayant 3 à 7 atomes de carbone ou un groupe aromatique ayant 6 ou 10 atomes de carbone, éventuellement substitué par de l'halogène, par un groupe alkyle ayant 1 à 2 atomes de carbone ou halogénoalkyle ayant 1 à 2 atomes de carbone, ou bien il représente un groupe hétéroaromatique ayant 5 ou 6 chaînons,

$R^4$ représente un atome d'hydrogène ou un groupe hydroxy, $R^5$ et $R^6$ sont identiques ou différents et représentent chacun un atome d'hydrogène ou un groupe alkyle ayant 1 à 4 atomes de carbone,

n représente des nombres valant 2 à 6, en particulier 2 à 4, et si $R^1$ représente un atome d'hydrogène,

leurs sels physiologiquement compatibles, pour un traitement préventif et un traitement curatif des maladies auxquelles le thromboxanne participe.

8. Procédé pour préparer des amides d'acides norbornane-carboxyliques et norbornène-carboxyliques de formule générale :

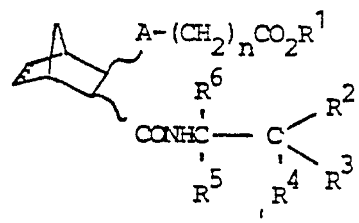

dans laquelle

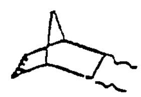

représente les structures partielles

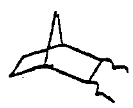

ou

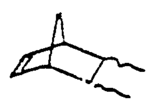

A représente un groupe

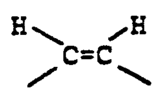

ou $-CH_2CH_2-$,

$R^1$ et $R^2$ représentent chacun un atome d'hydrogène ou un reste alkyle ayant 1 à 6, en particulier 1 ou 2, atomes de carbone,

$R^3$ représente un groupe alkyle ou alcényle ayant 1 à 8 atomes de carbone, éventuellement substitué par de l'halogène ou par un groupe alkyle ayant 1 à 2 atomes de carbone, ou il représente un groupe cycloalkyle et cycloalcényle ayant 3 à 7 atomes de carbone ou un groupe aromatique ayant 1 à 2 atomes de carbone ou halogénoalkyle ayant 1 à 2 atomes de carbone, ou bien il représente un groupe hétéroaromatique ayant 5 ou 6 chaînons,

$R^4$ représente un atome d'hydrogène ou un groupe hydroxy,

$R^5$ et $R^6$ sont identiques ou différents et représentent chacun un atome d'hydrogène ou un groupe alkyle ayant 1 à 4 atomes de carbone,

n représente des nombres valant 2 à 6, en particulier 2 à 4, et si $R^1$ représente un atome d'hydrogène, leurs sels physiologiquement compatibles,

caractérisé en ce qu'on réduit une lactone de formule générale (II)

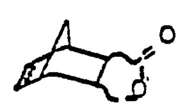

(II)

dans laquelle

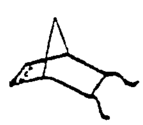

représente les structures partielles

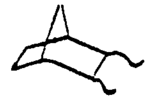

ou

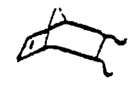

pour obtenir un lactol de formule générale (III)

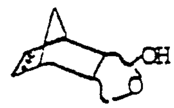

dans laquelle

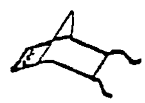

a le sens indiqué ci-dessus, et l'on fait réagir le lactol ainsi obtenu avec un sel de phosphonium de formule générale (IV) :

$$[(R^7)_3\overset{+}{P}(CH_2)_{n+1}\ CO_2H]X^-$$ (IV)

dans laquelle
$R^7$ représente un reste aryle,
X représente un halogène et
n représente un nombre dont la valeur est comprise entre 2 et 6, en présence d'une base pour obtenir un acide de formule générale (V) :

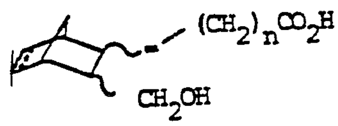 (V)

dans laquelle

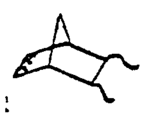

et n ont le sens précité et l'on fait ensuite réagir cet acide avec un alcool de formule générale (VI) :

$R^8OH$ (VI)

dans laquelle $R^8$ représente un reste alkyle ayant 1 à 6 atomes de carbone, pour obtenir l'ester de formule générale (VII) :

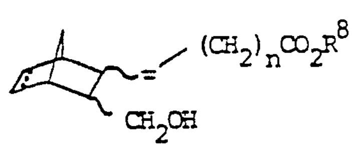 (VII)

dans laquelle

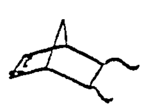

n et $R^8$ ont le sens indiqué ci-dessus, et si, dans la formule générale (I), A représente

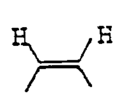

on oxyde ce dernier composé pour obtenir un acide de formule générale (VIII)

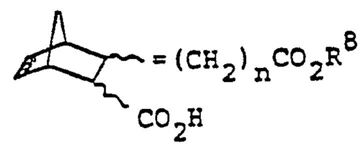

(VIII)

dans laquelle

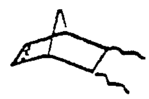

n et $R^8$ ont le sens indiqué ci-dessus, et l'on fait réagir l'acide ainsi obtenu, en le transformant en un ester activé, avec une amine de formule générale (IX) :

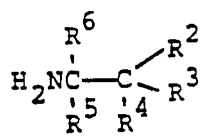

(IX)

dans laquelle

$R^2$ représente un atome d'hydrogène ou un reste alkyle ayant 1 à 6 atomes de carbone,

$R^3$ représente un groupe alkyle ou alcényle ayant 1 à 8 atomes de carbone, éventuellement substitué par de l'halogène ou par un groupe alkyle ayant 1 ou 2 atomes de carbone, ou il représente un groupe cycloalkyle ou cycloalcényle ayant 3 à 7 atomes de carbone ou un groupe aromatique ayant 6 ou 10 atomes de carbone (éventuellement substitué par de l'halogène, par un groupe alkyle ayant 1 ou 2 atomes de carbone ou par un groupe halogénoalkyle ayant 1 ou 2 atomes de carbone) ou il représente un noyau hétéroaromatique ayant 5 ou 6 chaînons,

$R^4$ représente un atome d'hydrogène ou un groupe hydroxy,

$R^5$ et $R^6$ sont identiques ou différents et ils représentent chacun un atome d'hydrogène ou un groupe alkyle ayant 1 à 4 atomes de carbone,

pour obtenir les amide-esters (I) ou, avec saponification subséquente, pour obtenir les amide-acides (I), ou si, dans la formule générale (I), A représente un groupe $-CH_2CH_2-$ et

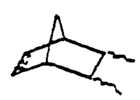

représente la structure partielle

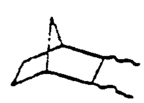

on hydrogène l'ester insaturé de formule générale (VI) pour obtenir l'ester de formule générale (X) :

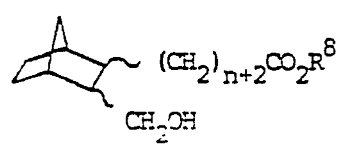

(X)

dans laquelle $R^8$ et n ont le sens indiqué ci-dessus, on le fait réagir pour obtenir un acide de formule générale (XI) :

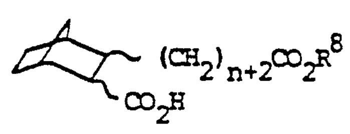

(XI)

dans laquelle $R^8$ et n ont le sens indiqué ci-dessus, et l'on fait réagir l'acide (XI) obtenu, éventuellement en le transformant en un ester activé, avec une amine de formule générale (IX) pour obtenir les amide-esters (I) ou, après saponification subséquente, pour obtenir les amides-acides (I).